# EUROPEAN PATENT APPLICATION

(11) **EP 3 311 739 A1**
(43) Date of publication of application: **25.04.2018**
(21) Application number: 17197327.4
(22) Date of filing: 19.10.2017
(51) Int. Cl.: A61B 5/103, A61B 5/11, A63B 24/00

(54) **ANALYSIS AND TREATMENT APPARATUS FOR MEDICAL, DIAGNOSTIC, SPORTS AND REHABILITATION USE**

(30) Priority: 20.10.2016 IT 201600105377
(71) Applicant: Bologna Isokinetic S.R.L., 40132 Bologna (IT)
(72) Inventor: DELLA VILLA, Francesco, 40135 Bologna (IT); DELLA VILLA, Stefano, 40135 Bologna (IT)
(74) Representative: Modiano, Micaela Nadia

(57) **Abstract**

An analysis and treatment apparatus (1) particularly for medical, diagnostic, sports and rehabilitation use, characterized in that it comprises:
- a room (2) provided with a planar and horizontal walking surface (3) and with at least one perimetric delimiting wall (4),
- at least one control and management element (5) associated with at least one memory unit,
- at least one load cell arranged below at least one portion (6) of the walking surface (3) in order to detect the force applied thereto by the patient (P) during the execution of predefined movements; the load cell is functionally associated with the control and management element (5),
- at least one image acquisition device (7, 8) for detecting images of a patient (P), the device (7, 8) being connected to the control and management element (5),
- at least one image display screen (9), which is arranged at the at least one perimetric wall (4) of the room (2), the at least one screen (9) is adapted to reproduce a sequence of images of the patient (P) substantially in life size in conditions chosen preferably among real-time, recorded, at real-time speed, at altered speed, with the addition of data strings, with the addition of graphic symbols and the like.

## Description

The present invention relates to an analysis and treatment apparatus particularly for medical, diagnostic, sports and rehabilitation use.

Many of the most common injuries and chronic conditions of the lower limb that affect an athlete can be traced back to alterations of the motor patterns that chronically (many movements for a long time) or acutely (a single movement) overload a region of the body, causing articular lesions.

Consequently, valid rehabilitation after injury and/or correct training that avoids problems of various kinds of the lower limbs entails repeating specific correct athletic gestures under the supervision of specialized personnel.

The number of repetitions required for correct acquisition of the correct movements on the part of a hypothetical patient (i.e., making these movements become instinctive) will be variable as a function of the abilities and the response to stimuli of the patient, as well as of the skill of the specialized personnel in transmitting the correct information to the patient.

It is therefore evident that each treatment (be it for rehabilitation, diagnosis or instruction, if an athlete is being trained in order to improve his/her athletic gestures) is heavily conditioned by the abilities of the specialized personnel that follows him/her, making a standard path almost impossible.

Moreover, during treatment the patient finds himself/herself performing complex athletic gestures, which require attention, and therefore has serious difficulties in following the verbal advice of the specialized personnel that follows him/her and is greatly hindered in observing his/her own movements in real time on the screen of a PC.

The aim of the present invention is to solve the problems described above, proposing an analysis and treatment apparatus particularly for medical, diagnostic, sports and rehabilitation use that allows to optimize the recovery of correct movement patterns, with the goal of improving the functional result of the patient and reducing the incidence of further injuries.

Within this aim, an object of the invention is to propose an analysis and treatment apparatus particularly for medical, diagnostic, sports and rehabilitation use that allows the patient to see and to detect clearly and straightforwardly the deficit that is present in his/her motion pattern.

A further object of the present invention is to provide an analysis and treatment apparatus particularly for medical, diagnostic, sports and rehabilitation use that has modest costs, is relatively simple to provide in practice and is safe in application.

This aim and these and other objects which will become better apparent hereinafter are achieved by an analysis and treatment apparatus particularly for medical, diagnostic, sports and rehabilitation use, characterized in that it comprises:
- a room provided with a planar and horizontal walking surface and with at least one perimetric delimiting wall;
- at least one control and management element associated with at least one memory unit;
- at least one load cell arranged below at least one portion of said walking surface in order to detect the force applied thereto by the patient during the execution of predefined movements, said load cell being functionally associated with said control and management element;
- at least one image acquisition device for detecting images of a patient, said device being connected to said control and management element;
- at least one image display screen, which is arranged at the at least one perimetric wall of said room, said screen being adapted to reproduce a sequence of images of the patient substantially in life size in conditions chosen preferably among real-time, recorded, at real-time speed, at altered speed, with the addition of data strings, with the addition of graphic symbols and the like.

Further characteristics and advantages of the invention will become better apparent from the description of a preferred but not exclusive embodiment of the analysis and treatment apparatus particularly for medical, diagnostic, sports and rehabilitation use according to the invention, illustrated by way of nonlimiting example in the accompanying drawings, wherein:
Figure 1 is a schematic perspective view of an analysis and treatment apparatus particularly for medical, diagnostic, sports and rehabilitation use according to the invention;

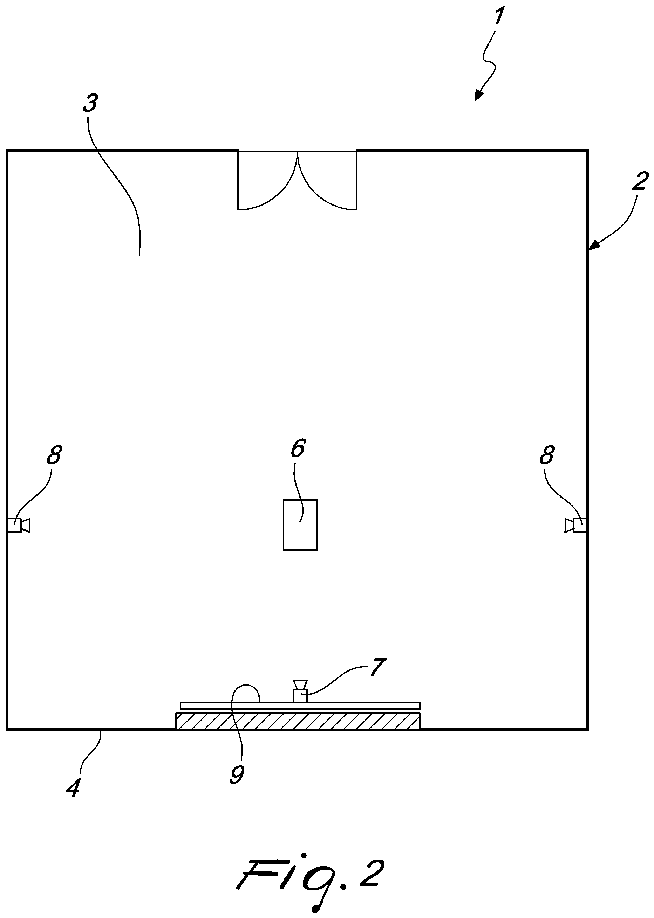

Figure 2 is a top view of the apparatus of Figure 1.

With particular reference to the figures cited above, the reference numeral 1 generally designates an analysis and treatment apparatus particularly for medical, diagnostic, sports and rehabilitation use.

The apparatus according to the invention is constituted essentially by a room 2 provided with a flat and horizontal walking surface 3.

The room can efficiently comprise also at least one perimetric delimiting wall 4.

Furthermore the apparatus comprises at least one control and management element 5 associated with at least one memory unit.

It is specified furthermore that at least one load cell is installed and arranged below at least one portion 6 of the walking surface 3 in order to detect the force applied thereto by the patient P during the execution of predefined movements. The load cell is also functionally associated with the control and management element 5.

It is specified that the apparatus 1 comprises furthermore at least one image acquisition device 7, 8 for detecting images of a patient P.

This device 7, 8 too is validly connected to the control and management element 5.

The apparatus 1 according to the invention is particularly efficient and user-friendly with respect to the patient P, since it comprises at least one image display screen 9, which is arranged at the at least one perimetric wall 4 of the room 2.

The screen 9 is adapted to reproduce a sequence of images of the patient P substantially in life size in conditions chosen preferably among real-time, recorded, at real-time speed, at altered speed, with the addition of data strings, with the addition of graphic symbols and the like.

In order to render the comprehension of the correct motor patterns and of any postural or athletic errors that affect the patient P particularly simple to the patient P himself/herself, the apparatus 1 uses a display screen 9 which has a length comprised between 2 m and 7 m and a height comprised between 1 m and 4 m.

In practice, the patient P is in front of a so-called video wall while he/she performs the coded exercises and movements, thus being able to observe in real time his/her own athletic gestures without being distracted from their execution (if he/she had to look at a small screen his/her attention would be partly transferred to the screen, sacrificing the athletic gesture, consequently compromising its execution).

It is not excluded to use display screens 9 of the curved type which generate a continuous image of the patient P or superimpose/arrange side by side different images which are mutually correlated in order to facilitate the comprehension of the correct motion patterns.

For example, the patient P might see centrally his/her own image in real time and see laterally three-dimensional simulations that perform the correct athletic gesture, thus being able to perform an easy visual comparison in real time.

It is not excluded, furthermore, to arrange further display screens 9 laterally with respect to the patient P: in this manner, during sequences of movements that provide for the patient P to turn about himself/herself, he/she can continue in any case to observe his/her own movements and optionally compare them with the reference movements or read the variations and/or displacements detected by the control and management element 5 with respect to the ideal movements.

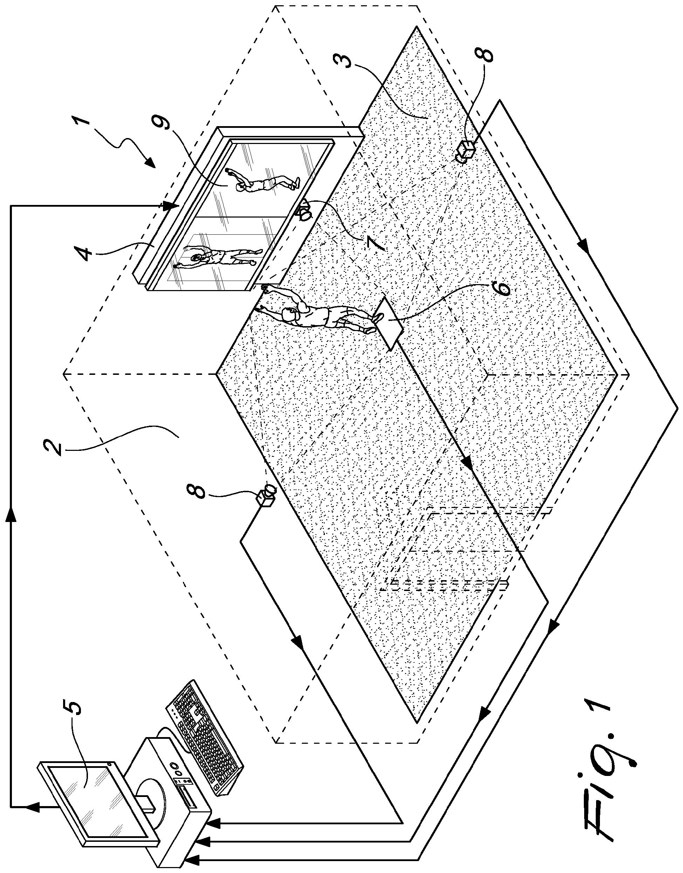

The choice of the reference parameters and of the type of activity to be performed can be selected by means of at least one user interface module (in the example shown in Figure 1, said module is integrated in the control and management element 5).

As already anticipated, the interface module is suitable to display and modify the results, processed by the control element 5, and to modify and define the images that can be displayed on the screen 9.

It is deemed useful to point out that the at least one image acquisition device 7, 8 is of the type preferably chosen from a high-speed video camera, an infrared video camera, an IP (Internet Protocol) camera, a two-lens video camera, a still camera, and the like.

The high-speed video camera is a particularly effective instrument, since it allows to analyze each individual movement, breaking it down into a highly detailed sequence of images. In this manner it is possible to detect the steps of the athletic gesture at which the greatest deviations with respect to the ideal motor pattern occur, making it easier for the patient to perform the corresponding corrections.

For other types of detection it can be useful to have an infrared video camera (or thermal camera) in order to detect the response of the body of the patient P to physical exercise and the heat dissipation capability. If it is necessary to study a correct training plan for athletes who must perform challenging and particular sports activities (such as marathons in the desert, polar expeditions, high altitude Alpine expeditions), it might be useful to detect which athletic gesture entails the lowest (or highest) dissipation of heat (in order to orient the training and athletic conditioning of the patient P).

It is specified that the apparatus 1 preferably comprises at least three image acquisition devices 7, 8.

Apparatuses 1 are provided in which there are image acquisition devices 7 in a front position with respect to the patient P or in the lateral position 8 again with respect to said patient.

However, the arrangement of further image acquisition devices located to the rear, above or below with respect to the patient P in order to increase the visual stimulus provided to the patient P, facilitating him/her in identifying motor and postural errors, is not excluded.

In particular, the lateral devices 8 are arranged on the lateral perimetric walls of the room 2, the rear device on the wall that is opposite with respect to the wall 4, the upper device on the ceiling of the room 2.

The optional device arranged below the patient P can be arranged below the walking surface 3, utilizing the portions 6 designed to accommodate the load cell.

By providing a portion 6 made of transparent material (for example made of methacrylate, polycarbonate, impact-resistant glass and the like) it is in fact possible to have an image acquisition device also below the patient P.

It is specified that the walking surface 3 that is preferably used comprises a covering paving of a shock-absorbing type in order to minimize impact traumas on the patient P during the execution, on the part of said patient, of a series of coded movements.

For example, the adoption of synthetic grass, of the type normally used to provide fields for soccer, rugby and the like, is provided.

In any case, the use of parquet, linoleum, technical substrates of the type used in athletics and the like, is not excluded.

In order to render the apparatus 1 efficient, it is specified that the memory unit of the control and management element 5 is a validly preset to store a plurality of data strings that correspond to profiles of the patient P.

Data of the type preferably chosen among an identification code, analysis, diagnosis, results of the tests and the like will be associated with each profile.

Furthermore, the memory unit also comprises an internal archive for collecting all the detections made, including a digital signature and a timestamp. This archive is therefore an archive for medical use of a complete type which can be shared within platforms for the study of new rehabilitation therapies, new diagnostic methods and for the generation of new specific training standards.

It is specified that the data strings comprise, stored in the memory unit, neuromotor patterns, motion patterns and the like: in particular, data related to the ideal conditions and comparison data related to the most frequent pathological conditions that can be observed in the patients P will be stored.

The present invention extends its protection also to a method for analysis and diagnosis of the medical type that consists of a series of consecutive steps.

First of all, it is necessary to place the patient P in a room 2 of an apparatus according to claim 1.

It is then necessary to ask the patient P to perform a series of coded movements.

During the execution of these coded movements on the part of the patient P, it is necessary to detect, by means of the at least one load cell, the intensity, direction and variations of the force applied by the patient P. It is then necessary to transfer these data to a control and management element 5.

Likewise, it is necessary to detect sequences of images, by means of at least one image acquisition device 7, 8, again referred to the patient P while he/she is performing the series of coded movements, followed by the transfer of the data to a control and management element 5.

One then proceeds with the comparison of the data acquired in the preceding steps with predefined reference data strings contained within an adapted memory unit controlled by the control and management element 5.

By virtue of the presence of the display screen 9 it is therefore possible (even in real time) to display on a screen the acquired data and their deviation with respect to the reference data, highlighting said deviation directly on the sequences of acquired images of the patient P.

As already mentioned earlier, the comparison data strings used shall comprise, stored in the memory unit, neuromotor patterns, motion patterns, and the like of a type which is coded and known in the scientific literature.

The patient P can thus benefit from an immediate feedback of his/her own motor pattern on the large screen 9, viewing his/her own figure in life-size, in order to understand in the best way the deficits observed in front and lateral viewing (or optionally also rear, lower or upper viewing) during the dynamic movements of the analysis test.

The Motion Analysis Test (MAT) also can be performed within the room 2 allowing to detect altered movement patterns, which often cause injuries, and correcting them with specifically dedicated neuromotor training sessions before returning to the execution of sport-specific movements.

On the basis of the results obtained from the Motion Analysis Test (MAT), the specialist physician establishes a customized biomechanical rehabilitation program, with the goal of correcting the motion dysfunction and of retraining motor coordination, but also of accelerating rehabilitation and reducing the risk of reinjury after return to sports.

The apparatus 1 according to the invention therefore allows to perform activities for rehabilitation and functional recovery after muscle-skeletal injuries, with particular reference to the optimization of complex movement patterns and to the prevention of any repetitions of injury.

The apparatus 1 furthermore allows to implement primary prevention of injury in the healthy athlete.

Advantageously, the present invention solves the problems described previously, proposing an analysis and treatment apparatus 1 particularly for medical, diagnostic, sports and rehabilitation use that allows to optimize the recovery of correct motor patterns, with the aim of improving the functional result of the patient P and reducing the incidence of further injuries.

Positively, the apparatus 1 according to the invention allows the patient P to view and identify clearly and immediately the deficit that is present in his/her own motor pattern.

Validly, the present invention can be provided in a relatively simple manner, bearing especially modest costs: these characteristics make the apparatus 1 according to the invention an innovation of assured application.

The invention thus conceived is susceptible of numerous modifications and variations, all of which are within the scope of the appended claims; all the details may furthermore be replaced with other technically equivalent elements.

In the examples of embodiment shown, individual characteristics, given in relation to specific examples, may actually be interchanged with other different characteristics that exist in other examples of embodiment.

In practice, the materials used, as well as the dimensions, may be any according to the requirements and the state of the art.

The disclosures in Italian Patent Application no. 102016000105377 (UA2016A007500), from which this application claims priority, are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. An analysis and treatment apparatus particularly for medical, diagnostic, sports and rehabilitation use, **characterized in that** it comprises:
- a room (2) provided with a planar and horizontal walking surface (3) and with at least one perimetric delimiting wall (4);
- at least one control and management element (5) associated with at least one memory unit;
- at least one load cell arranged below at least one portion (6) of said walking surface (3) in order to detect the force applied thereto by the patient (P) during the execution of predefined movements, said load cell being functionally associated with said control and management element (5);
- at least one image acquisition device (7, 8) for detecting images of a patient (P), said device (7, 8) being connected to said control and management element (5);
- at least one image display screen (9), which is arranged at the at least one perimetric wall (4) of said room (2), said screen (9) being adapted to reproduce a sequence of images of the patient (P) substantially in life size in conditions chosen preferably among real-time, recorded, at real-time speed, at altered speed, with the addition of data strings, with the addition of graphic symbols and the like.

2. The analysis and treatment apparatus according to claim 1, **characterized in that** said display screen (9) has a length comprised between 2 m and 7 m and a height comprised between 1 m and 4 m.

3. The analysis and treatment apparatus according to claim 1, **characterized in that** it comprises at least one user interface module, said interface module being suitable to display and modify the results, processed by said control and management element (5), and to modify and define the images that can be displayed on said at least one screen (9).

4. The analysis and treatment apparatus according to claim 1, **characterized in that** said at least one image acquisition device (7, 8) is of the type chosen preferably among a high-speed video camera, an infrared camera, an IP (Internet Protocol) video camera, a two-lens video camera, a still camera and the like.

5. The analysis and treatment apparatus according to claim 1, **characterized in that** it comprises at least three image acquisition devices (7, 8), said devices being preferably arranged in a position, with respect to the patient (P), which is chosen among frontally (7), laterally (8), upward, to the rear and below.

6. The analysis and treatment apparatus according to claim 1, **characterized in that** said walking surface comprises a covering paving of the shock-absorbing type in order to minimize impact traumas on the patient (P) during the execution, on the part of said patient (P), of a series of coded.

7. The analysis and treatment apparatus according to one or more of the preceding claims, **characterized in that** said memory unit stores a plurality of strings of data which correspond to patient profiles (P), data of the type chosen preferably among an identification code, an anamnesis, a diagnosis, the results of tests and the like being associated with each profile.

8. The analysis and treatment apparatus according to one or more of the preceding claims, **characterized in that** said memory unit comprises an internal archive for collecting all the detections performed, comprising a digital signature and a timestamp.

9. The analysis and treatment apparatus according to one or more of the preceding claims, **characterized in that** said data strings comprise, stored in said memory unit, neuromotor patterns, movement patterns and the like.

10. A method for analysis and diagnosis of the medical type, which consists of the following steps:
a) placing a patient (P) in a room (2) of an apparatus (1) according to claim 1;
b) asking the patient (P) to perform a series of coded movements;
c) detecting, by means of the at least one load cell, the intensity, direction and variations of the force applied by the patient (P) during the execution of the series of coded movements, on the respective portion (6) of said walking surface (3), and transferring said data to a control and management element (5);
d) detecting sequences of images, by means of an image acquisition device (7, 8), of the patient (P) during the execution of the series of coded movements, and transferring said data to a control and management element (5);
e) comparing the data acquired in the preceding steps with predefined reference data strings contained within an adapted memory unit controlled by said control and management element (5);
f) displaying on at least one screen (9) the acquired data and their deviation with respect to the reference data, highlighting said deviation directly on the sequences of acquired images of the patient (P).

11. The method according to claim 10, **characterized in that** said data strings comprise neuromotor patterns, movement patterns,and the like stored in said memory unit.
